# EUROPEAN PATENT SPECIFICATION

(11) **EP 2 779 002 B1**
(45) Date of publication and mention of the grant of the patent: **04.07.2018**
(21) Application number: 14159539.7
(22) Date of filing: 13.03.2014
(51) Int. Cl.: G06F 19/00, A61G 7/00

(54) **Hospital bed for receiving data from thin patch wireless sensors**
Krankenhausbett zum Empfang von Daten von drahtlosen Sensoren in dünnem Pflaster
Lit d'hôpital pour recevoir les données provenant de capteurs sans fil à plaque mince

(30) Priority: 15.03.2013 US 201361791019 P
(43) Date of publication of application: 17.09.2014
(73) Proprietor: Hill-Rom Services, Inc., Batesville, IN 47006-9167 (US)
(72) Inventor: Receveur, Timothy, Joseph, Guilford, IN Indiana 47022 (US); Lachenbruch, Charles, A, Lakeway, TX Texas 78734 (US); Sauser, Frank, E, Cincinnati, OH Ohio 45248 (US)
(74) Representative: Findlay, Alice Rosemary

(56) References cited:
- WO-A1-2012/108935
- WO-A2-2009/135081
- US-A1- 2005 102 167

## Description

The present disclosure is related to a patient apparatus that includes a wireless communication system that is operable to gather data from patient sensors. More specifically, the present disclosure is related to a communication system for a patient support apparatus that is operable to inductively power sensors coupled to a patient to periodically gather data from the sensors.

Patient support apparatuses such as hospital beds, for example, have become more sophisticated including the implementation of communications networks between various modules of the patient support apparatus. In a hospital setting, the identification and location of a particular patient is often associated with a particular patient support apparatus. Patient support apparatuses may include a unique identifier which identifies the particular patient support apparatus to external systems such as a hospital information system, for example. It is important that the appropriate patient be associated with a patient support apparatus so that the hospital information system can track the patient and maintain the patient's electronic medical record.

Because of the close proximity of a patient to a patient support apparatus, the patient support apparatus may serve as a data accumulator regarding information about the patient and peripheral devices being used to monitor or treat the patient. Patient comfort is an important aspect for patient recovery. This has resulted in a movement to reduce the obtrusiveness of monitoring equipment and sensors. The reduction in obtrusiveness is generally in tension with the need to properly identify the information provided and associate that information with the proper patient.

US 2005/0102167 discloses a method of automating some of the tasks requiring continuous data collection at the patient bedside in a hospital. These tasks include provisioning of the IV pumps or other fluid infusion pumps, feed pumps, oxygen delivery systems, gathering, recording, storing, and analyzing signals from ECG machine or pulse oxymeter or any other medical device. Wireless transceiver modules are used which are connected to the data ports on the medical instrument to gather the data and transmit the data to a wireless access point. Protocols to identify the patient, care provider, medicine, equipment, and treatment are described. Use of an external means for verifying the identity of the medical device and the medicine is also described.

According to the present invention, a method for transferring medical data from a wireless sensor to a health information system using a patient support apparatus comprises wirelessly transferring, with the patient support apparatus, a supply of power to the wireless sensor to initiate operation of the wireless sensor. The method further comprises while wirelessly transferring the supply of power to the wireless sensor, receiving, with the patient support apparatus, the medical data from the wireless sensor. The method still further comprises determining, on the patient support apparatus, an identity of the wireless sensor. The method still yet further comprises determining, with the patient support apparatus, a nature of the received medical data. The method also further comprises associating the received medical data with the patient support apparatus. The method also yet further comprises transmitting, from the patient support apparatus, the received medical data and data identifying the associated patient support apparatus to the health information system subsequent to determining the nature of the received medical data.

In some embodiments, receiving the medical data comprises receiving the medical data from wireless communication circuitry.

In some embodiments, receiving the medical data from the wireless sensor is in response to establishing a wireless communication connection between the patient support apparatus and the wireless sensor.

In some embodiments, receiving the medical data is in response to transmitting, from the patient support apparatus, a request for the medical data.

In some embodiments, determining the identity of the wireless sensor comprises performing, on the patient support apparatus, a handshaking protocol with the wireless sensor.

In some embodiments, performing the handshaking protocol comprises receiving, with the patient support apparatus, a unique identifier of the wireless sensor from the wireless sensor.

In some embodiments, determining the nature of the received medical data comprises receiving identifying information associated with the medical data from the wireless sensor.

In some embodiments, transmitting the received medical data and the data identifying the associated patient support apparatus comprises transmitting an alert message to the hospital information system.

In some embodiments, transmitting the alert message comprises transmitting an alert message to a nurse's station using a nurse call cable.

In some embodiments, transmitting the alert message comprises wirelessly transmitting the alert message.

In some embodiments, the method further comprises analyzing, on the patient support apparatus, the received medical data to determine whether a health condition is met, the health condition and associated medical data criteria having been pre-established on the patient support apparatus.

In some embodiments, the method further comprises performing a function using the patient support apparatus in response to the received medical data satisfying the health condition.

In some embodiments, performing the function comprises at least one of automatically performing a mechanical function and transmitting an alarm to the hospital information system.

In some embodiments, the method further comprises transmitting, from the patient support apparatus to the wireless sensor, instructions to update the wireless sensor in response to receiving the instructions from the hospital information system.

According to another aspect of the present invention a controller of a patient support apparatus comprises a processor and a memory having stored therein a plurality of instructions. When executed by the processor the instructions cause the controller to wirelessly transfer a supply of power to a wireless sensor to initiate operation of the wireless sensor. When executed by the processor the instructions also cause the controller to receive medical data from the wireless sensor. When executed by the processor the instructions also cause the controller to determine an identity of the wireless sensor. When executed by the processor the instructions also cause the controller to determine a nature of the received medical data. When executed by the processor the instructions also cause the controller to associate the received medical data with the patient support apparatus. When executed by the processor the instructions also cause the controller to transmit the received medical data to a health information system.

In some embodiments, the wireless sensor comprises wireless communication circuitry including an inductor for receiving wireless power.

In some embodiments, the plurality of instructions cause the controller to receive the medical data from the wireless sensor in response to establishing a wireless communication connection between the patient support apparatus and the wireless sensor.

In some embodiments, the plurality of instructions cause the controller to receive the medical data in response to a request for the medical data from the patient support apparatus.

In some embodiments, the plurality of instructions cause the controller to determine the identity of the wireless sensor by performing a handshaking protocol with the wireless sensor.

In some embodiments, the handshaking protocol comprises a transmission of a unique identifier of the wireless sensor from the wireless sensor to the controller.

In some embodiments, the plurality of instructions cause the controller to determine the nature of the received medical data by receiving identifying information associated with the medical data from the wireless sensor.

In some embodiments, the received medical data comprises an alert message.

In some embodiments, the plurality of instructions cause the controller to transmit the alert message to a nurse's station using a nurse call cable.

In some embodiments, the plurality of instructions cause the controller to wirelessly transmit the alert message.

In some embodiments, the plurality of instructions further cause the controller to analyze the received medical data to determine whether a pre-established health condition is met.

In some embodiments, the plurality of instructions cause the patient support apparatus to perform a function in response to the received medical data satisfying the pre-established health condition.

In some embodiments, the function comprises at least one of a mechanical operation and transmitting an alarm to the hospital information system.

In some embodiments, the plurality of instructions cause the controller further to transmit to the wireless sensor instructions to update the wireless sensor in response to receiving the instructions from the hospital information system. The invention will now be further described by way of example with reference to the accompanying drawings, in which:
Fig. 1 is diagrammatic representation of the structure of a patient support apparatus that is connected to a network in a hospital and in communication with a passive wireless sensor through a wireless connection;
Fig. 2 is a diagrammatic side view of a first embodiment of the passive wireless sensor of Fig. 1;
Fig. 3 is a diagrammatic top plan view of a second embodiment of a passive wireless sensor;
Fig. 4 is a diagrammatic top plan view of a third embodiment of a passive wireless sensor;
Fig. 5 is a diagrammatic top plan view of a fourth embodiment of a passive wireless sensor;
Fig. 6 is the portion of a flowchart describing an operational process of a controller of the patient support apparatus of Fig. 1;
Fig. 7 is the remaining portion of the flowchart of Fig. 6;
Fig. 8 is a flowchart representation of a subroutine of the operational process of Figs. 6 and 7 describing how data received from an wireless sensor is identified by the controller;
Fig. 9 is a flowchart representation of a subroutine of the operational process of Figs. 6 and 7 describing how data is analyzed to determine if a predetermined condition established in the controller is met by the wireless sensor;

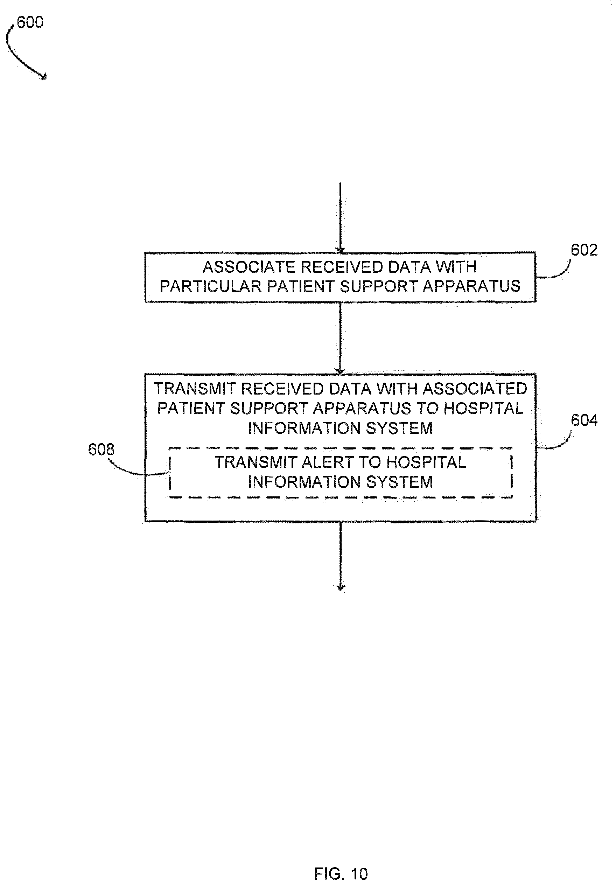

Fig. 10 is a flowchart representation of a subroutine of the operational process of Figs.6 and 7 describing how data from the wireless sensor is associated with the patient support apparatus; and
Fig. 11 is a perspective view of a patient support apparatus that includes a number of readers for reading data from passive wireless sensors.

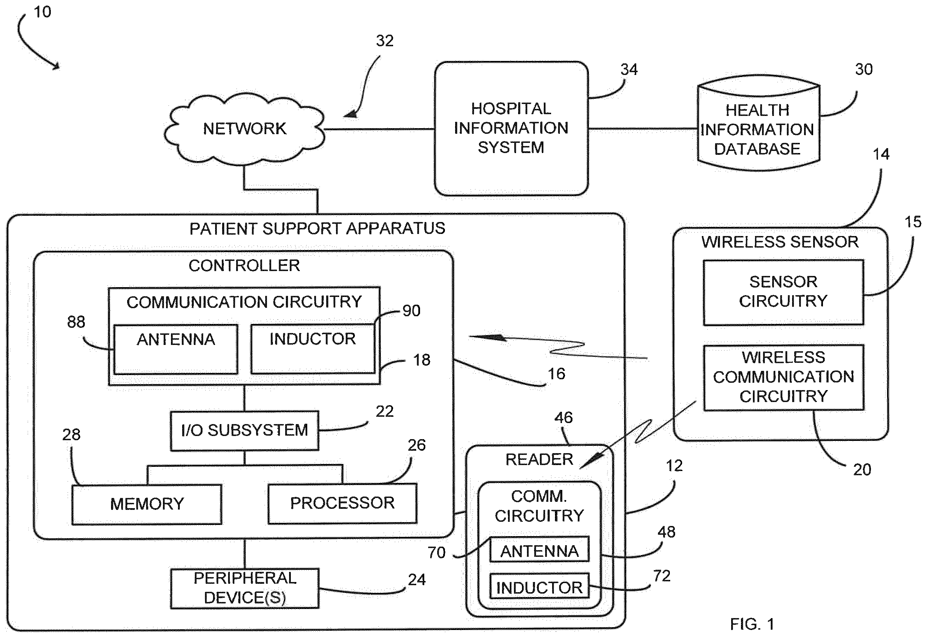

A system 10 for gathering information from a wireless sensor 14 and transferring the information to a health information database 30 includes a patient support apparatus 12 that has communication circuitry 18 that is operable to communicate with communication circuitry 20 of the wireless sensor 14 as suggested by Fig. 1. The illustrative wireless sensor 14 includes sensor circuitry 15 that is operable to determine a vital sign of a patient.

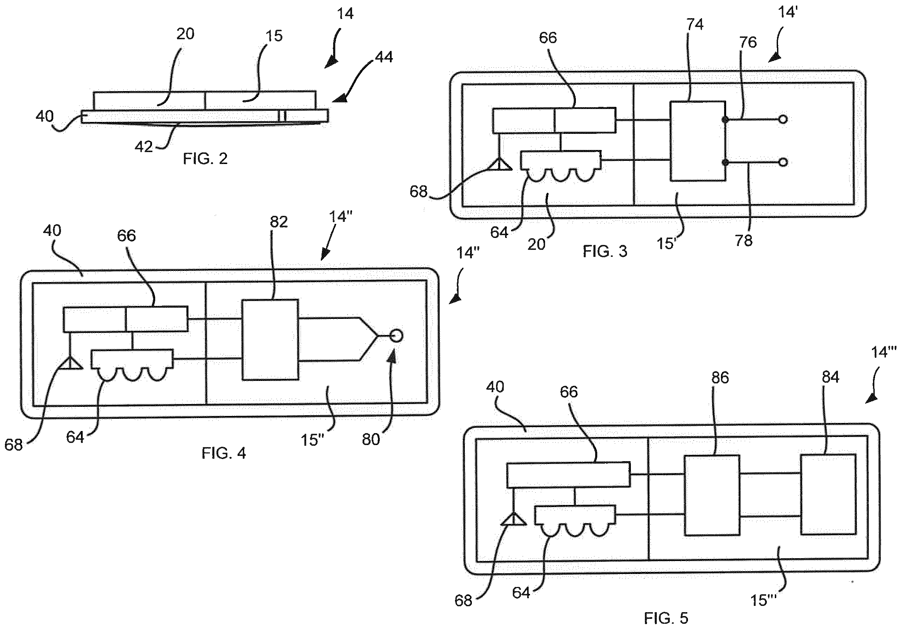

As shown in Fig. 2, the sensor 14 comprises a thin patch of polyester 40, with an adhesive 42 that secures the sensor 14 to the skin of a patient. The sensor 14 further includes a thin-film circuit 44 that is mounted to the polyester patch 40, the thin-film circuit 44 including the communication circuitry 20 and the sensor circuitry 15. The details of various embodiments of thin-film circuits will be discussed in further detail below. In some embodiments, the wireless sensor 14 may be a passive device that is unpowered and is operable to be energized inductively. In other embodiments, the wireless sensor 14 may have a small charge source, such as a battery, for example, to power the thin-film circuit 44. Various embodiments of the wireless sensor 14 may be configured for detecting one more biophysical characteristics of a patient such as heart rate, temperature, respiration rate, blood pressure, pulse oximetry, electrocardiographic (EKG) information, electroencephalographic (EEG), information, muscle movement, or other similar information. Other embodiments of the wireless sensor 14 may be configured to detecting one or more biochemical characteristics of a patient such as pathogens and/or chemical markers, chemical or biological markers, biomarkers, or the like. In one embodiment, the wireless sensor 14 is a monitoring sensor available from MC10, Inc. of Cambridge, Massachusetts. When detecting biochemical characteristics, the sensor circuitry 15 may include metal oxide type semi-conductor sensors and surface acoustic wave devices. In another embodiment, protein based biosensors such as odarant binding proteins, sensory appendage proteins, odorant or gustatory receptors, serpentine receptors and / or odarant degrading enzymes may be used along with electrochemical transducers to generate an electrical signal in response to detection of a chemical. In some embodiments, the sensor circuitry 15 may monitor for the presence of biochemical markers of wound development such as IL-1α and Creatine Phosphokinase (CPK).

Utilizing the close proximity of the wireless sensor 14 to the patient support apparatus 12, the identity of the patient to which the wireless sensor 14 is applied allows the association of the person with the patient support apparatus 12. Based on this association, the data from the wireless sensor 14 is transmitted by the patient support apparatus 12 to a network 32 that couples the patient support apparatus 12 to a hospital information system 34, utilizing the information about the person to associate the data with the person's specific electronic medical record. In other embodiments, the network 32 may be coupled to a traditional nurse call system.

The wireless sensor 14 includes the wireless communication circuitry 20. The patient support apparatus 12 includes a controller 16 which includes the communication circuitry 18 that acts as an interface to the wireless sensor 14. The controller 16 also includes an I/O subsystem 22 coupled to the communication circuitry 18 as well as a processor 26 and a memory device 28. The processor 26 is operable to use instructions stored in memory 28 to operate the I/O subsystem 22 which controls communication circuitry 18 as well as communication with the network 32. The controller 16 is in communication with peripheral devices of the patient support apparatus, such as peripheral device 24 shown in Fig. 1. Peripheral device 24 may be any of a number of subsystems of a patient support apparatus known in the art. For example if patient support apparatus 12 is embodied as a hospital bed, peripheral device 24 may include any one of a scale system, side rail position monitoring system, a brake mechanism monitoring system, a bed position monitoring system, a patient position monitoring system including bed exit detection capability, or a therapy device such as a therapeutic mattress, for example. In general, peripheral device 24 may be embodied as any subsystem or device that monitors a patient condition, monitors and operating condition of the patient support apparatus, controls and operating condition of the patient support apparatus, or provides therapy to patient supported on the patient support apparatus 12.

It is contemplated that the controller 16 may be programmed to operate as a universal interface capable of communicating with any of a number of different wireless sensors 14. During normal operation, controller 16 will regularly attempt to initiate communication with a wireless sensor such that any wireless sensor within the operating range of the communication circuitry 18 of the controller 16 may be detected and engaged by the controller 16.

The patient support apparatus 12 may also include one or more separate readers 46 that are coupled to the controller 16, the readers including communication circuitry 48 that communicates with the I/O subsystem 22 to share information with the controller 16. As shown in Fig. 11, separate readers 46₁, 46₂, 46₃, 46₄, and 46₅ are positioned on a surface 50 of a mattress 52 of the patient support apparatus 12. Another reader 46₆ is positioned in a headboard 54. Still another reader 46₇ is positioned in a footboard 56. Additional readers 46₈, 46₉, 46₁₀, and 46₁₁ are positioned in respective side rails 58, 60, 62, and 64. Each of the readers 46₁,- 46₁₁ are optional and may be included depending on the needs of a particular application.

The communication circuitry 18 includes an antenna 88 that receives the wireless signal from the sensor 14 and an inductor 90 that is operable to generate a magnetic field that generates a current in an inductor 64 of the communication circuitry 20 of the wireless sensor 14. The communication circuitry 20 also includes a power circuit 66 that is operable to convert the current generated in the inductor 64 to power the communication circuitry 20 and sensor circuitry 15 of the wireless sensor 14. The communication circuitry 20 also includes an antenna 68 that transmits signals from the communication circuitry 20 to the communication circuitry 18 of the controller.

Each reader 46₁,- 46₁₁ includes a separate structure similar to the communication circuitry 18 of the controller 16, but are spaced apart from the controller 16 and communicate through the I/O subsystem 22. Referring again to Fig. 1, the reader 46 includes an antenna 70 and an inductor 72 that are operable to communicate with a wireless sensor 14. Because the wireless sensor 14 is a passive device, the controller 16 is operable to cause the inductor 72 of a particular reader46₁,- 46₁₁ to generate a magnetic field on an intermittent basis. When the magnetic field is generated, a wireless sensor 14 in range of the reader 46 will receive power and begin to operate the sensor circuitry 15 and communication circuitry 20.

In one embodiment shown in Fig. 3, a wireless sensor 14' includes sensory circuitry 15' that includes logic 74 and a pair of leads 76 and 78 that are operable to measure a potential over the patient's skin. The polyester patch 40 is formed with openings that allow the leads 76 and 78 to contact a patient's skin, while keeping the remainder of the circuitry isolated from the patient's skin. The leads 76 and 78 and logic 74 are configured to measure electrical activity such as a heart rate.

In another embodiment, a wireless sensor 14" includes a thermocouple 80 in communication with logic 82 as shown in Fig. 4. The thermocouple 80 is used to determine the temperature of a patient's skin. In still another embodiment shown in Fig. 5, wireless sensor 14'" includes an electrochemical cell 84 that includes a chemistry that is responsive to an analyte that is introduced to the electrochemical cell 84. The output of the electrochemical cell 84 is used by logic 86, the logic 86 communicating the results of the analysis to the communication circuitry 20'" to be transmitted to the controller 16. The analyte is responsive to the chemistry such that an enzymatic reaction occurs generating a current in the electrochemical cell 84 that is proportional to the quantity of analyte, such as CPK, for example. It should be understood that any of a number of other biophysical or biochemical sensing structures may be implemented in the wireless sensors disclosed herein.

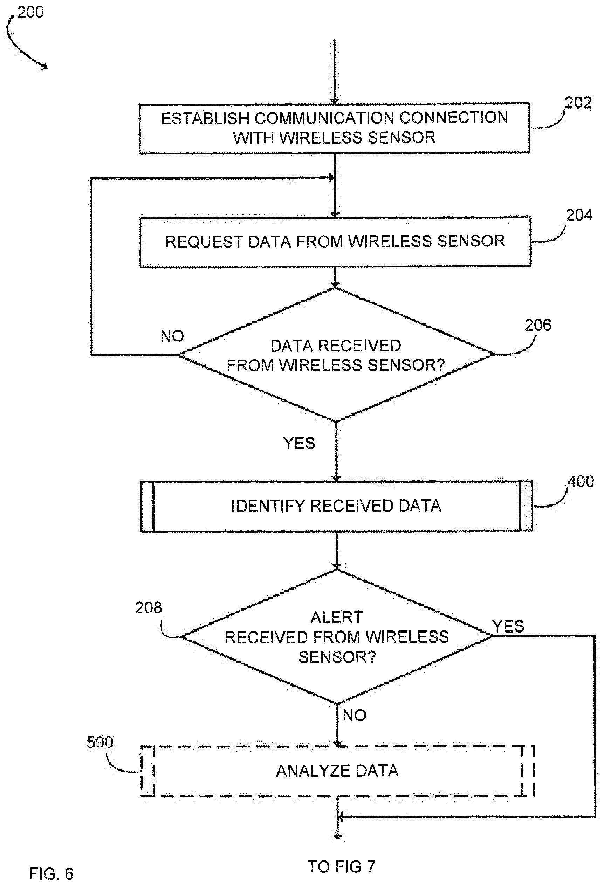

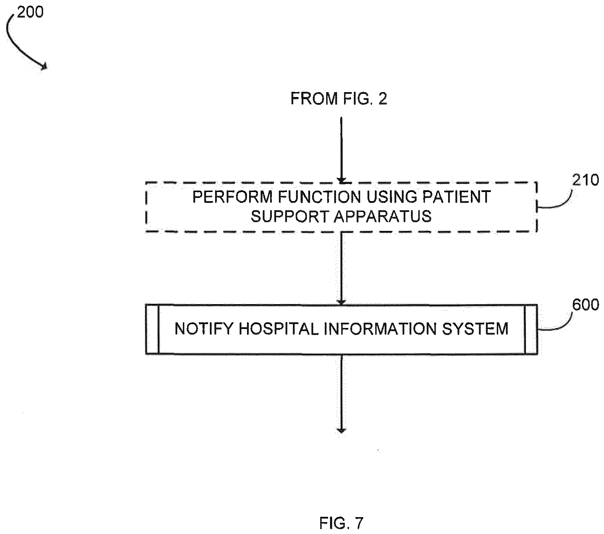

A process 200 shown in Figs. 6-7 provides an overview of the various actions the controller 16 may take in relation to the wireless sensor 14. Process steps shown in phantom indicate that the particular process step is optional as will be discussed in further detail below. At the initial process step 202 the controller 16 establishes a communication connection with the wireless sensor 14 with the communication circuitry 18 of controller 16 operating to initiate communications with the wireless communication circuitry 20 of the wireless sensor 14.

Process 200 proceeds to process step 204 where the controller 16 requests data from the wireless sensor 14. At process step 204 controller 16 will at least request sufficient data from the wireless sensor 14 to establish the identity of the person to which the wireless sensor 14 is applied. Other data may be also requested from the wireless sensor 14, such as operating a device serial number and sensor data from the sensor circuitry 15. In embodiments where the wireless sensor 14 includes a charge source, such as a battery, for example, it is contemplated that the request for data from the wireless sensor 14 will be intermittent to limit the power consumption of the wireless sensor 14.

Process 200 proceeds to decision step 206 where the controller 16 evaluates whether data has been received from the wireless sensor 14. Received data may be in response to a request made at process step 204. If no data has been received, process 200 returns and continues to monitor for received data. If data is received from the wireless sensor 14, process 200 proceeds to a subroutine 400 that is used to identify the received data. Subroutine 400 will be discussed in further detail below.

Process 200 then proceeds to decision step 208 to determine whether data that has been received from the wireless sensor 14 should trigger an alert. Alerts may be triggered by a malfunction of the wireless sensor 14 or a negative physiological condition of the patient as detected by the wireless sensor 14.

If no immediate alert is necessary, then process 200 proceeds to subroutine 500 in which the data that is provided is analyzed by the controller 16 as will be discussed in further detail below. It should be understood that an output of subroutine 500 may include analysis of data that when analyzed indicates an intervention is required.

Process 200 proceeds to optional process step 210 in which the controller 16 may change an operating parameter of the patient support apparatus 12. This may include the modification of the operation of any peripheral device 24. For example, if data received from the wireless sensor 14 indicates that a patient is in cardiac arrest or respiratory distress, controller 16 may automatically move one or more patient support apparatus member to a position which assists a caregiver in intervening with the patient. In the case of cardiac arrest, controller 16 may move the bed to a CPR position in anticipation of a caregiver having to perform CPR on the patient. If data from the wireless sensor 14 indicates that the patient is in respiratory distress, for example, controller 16 may move a member of the patient support apparatus 12 to position which increases the angle of incline of the patient's upper body so that the patient is better able to breathe. In still another example, if the wireless sensor 14 is operable to detect a patient's blood pressure, and blood pressure is determined to be low, controller 16 may move the patient support apparatus members such that the patient's legs are elevated. If the peripheral device 24 is a therapeutic mattress, controller 16 may change the operation of the therapeutic mattress, for example, to increase a relative level of pulmonary therapy.

Process 200 then proceeds to subroutine 600 were the controller 16 is operable to notify the hospital information system 34 of information related to the wireless sensor 14. As will be discussed in further detail below the subroutine 600 is operable to associate information regarding the wireless sensor 14 with the person to which the wireless sensor 14 is applied and the patient support apparatus 12 so that the information may be properly placed in the health information database 30 and associated with the particular persons electronic medical record.

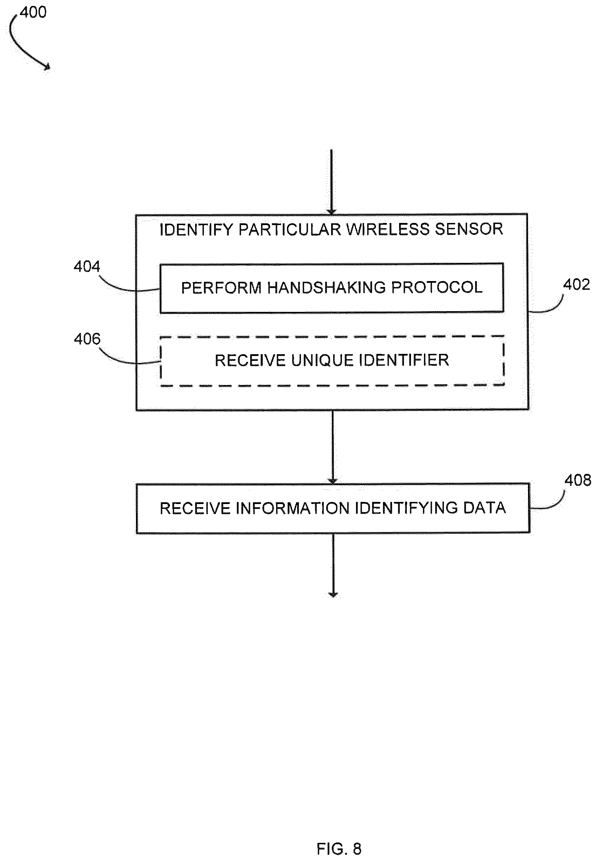

Referring now to Fig. 8, subroutine 400 includes a first process step 402 in which the controller 16 is operable to identify the wireless sensor 14 as a particular wireless sensor. A sub-process step 404 is a handshaking protocol that is used to confirm the compatibility of the controller 16 as an interface of the wireless sensor 14. In identifying the particular wireless sensor 14, the controller 16 may optionally receive a unique identifier from the wireless sensor 14 as indicated by optional process step 406. Once the relationship between the particular wireless sensor 14 and the controller 16 is established, the controller 16 receives identifying data from the particular wireless sensor 14. This data may include an explicit patient identification number or other information that associates the wireless sensor 14 with a particular patient. As noted above, once subroutine 400 is completed process 200 continues to the decision step 208 discussed above.

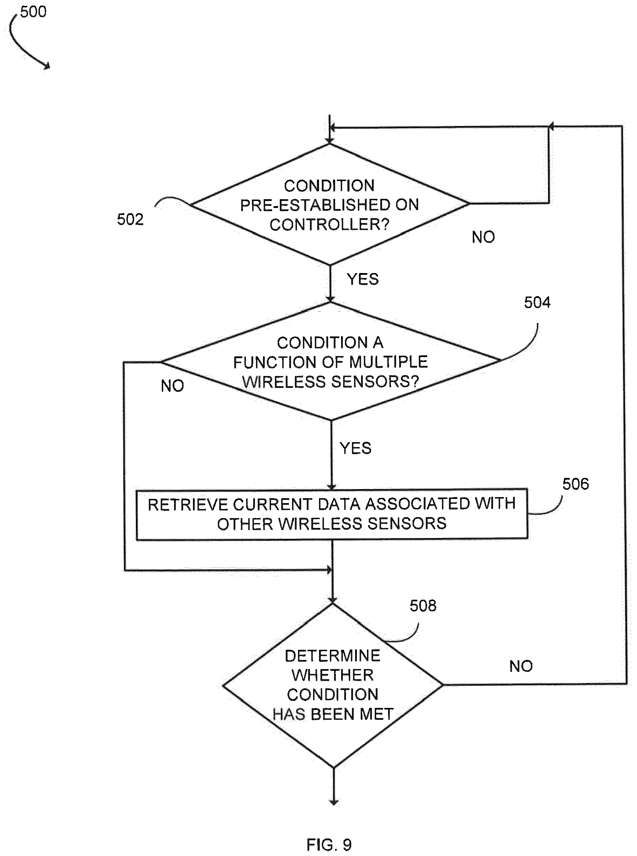

As discussed above subroutine 500, shown in Fig. 9, includes a first decision step 502 where it is determined if a condition is pre-established on the controller 16 that is related to data from the particular wireless sensor 14. If no such condition is pre-established on the controller 16, then the subroutine 500 loops back and continues to monitor for data from a wireless sensor that does have a condition that is pre-established on the controller 16. If it is determined that a health condition or device malfunction condition that is pre-established on controller 16 does relate to data from the particular wireless sensor 14, subroutine 500 proceeds to decision step 504 where the condition is further evaluated to determine if it is a function of a single wireless sensor, or of multiple wireless sensors. If it is determined at decision step 504 that the condition is dependent on only the particular wireless sensor 14, subroutine 500 progresses to decision step 508 to determine whether the condition has been met. If the condition has not been met, then subroutine 500 loops back and continues to monitor for relevant data. If the condition has been met as evaluated at decision step 508, then process 200 proceeds to either optional process step 210 or subroutine 600 as described above.

If at decision step 504 for it is determined that the condition is a function of multiple wireless sensors, then subroutine 500 proceeds to process step 506 where data from other wireless sensors or peripheral devices 24 is retrieved so that all of the data from each of the relevant devices may be evaluated to determine whether a condition has been met at decision step 508. Such a situation may exist when, for example, data from a wireless sensor 14 that acts as a blood pressure sensor is compared to data from a wireless sensor 14 that acts as an EKG sensor and the combined conditions of the two indicate an unsafe condition or a level of distress in the patient that may not be identified by either device independently. In some embodiments, the information from the wireless sensor 14 may be compared to information from one of the peripheral devices 24 to determine if a particular condition has been met. For example, a peripheral device 24 may be a traditional blood pressure monitor and a wireless sensor 14 may be an EKG sensor and the condition may involve evaluating data from both the blood pressure monitor and a cardiac defibrillator to determine whether the condition has been met.

When subroutine 600 is invoked, the data from the wireless sensor 14 is associated with a unique identifier for the particular patient support apparatus 12. A unique identifier for the patient support apparatus 12 is maintained in memory 28 and is associated with data from the wireless sensor 14 using methods known in the art. Subroutine 600 then proceeds to process step 604 where the associated data is transmitted to the hospital information system 34 over the network 32 to be stored in the health information database 30. Process step 604 includes an optional process step 608 that includes transmitting alert information to the hospital information system 34 if an alert condition is generated by the wireless sensor 14. If the condition has been determined to be met in subroutine 500, that information may also be transferred over the network 32 to the hospital information system 34. In other embodiments, the I/O subsystem 22 may also transmit the alert condition to a nurse's station over a traditional nurse call cable.

Information may be transferred over the network 32 to the hospital information system 34 by the controller 16 in real time, or may be stored in memory 28 and transferred to the network 32 on an intermittent basis. In still other embodiments, when the information is stored on the controller 16, the hospital information system 34 may be operable to query the controller 16 to receive the most recent information stored by controller 16 in memory 28. Controller 16 may combine and associate information from peripheral devices 24 as well as wireless sensor 14 so that all of the information may be transferred to the hospital information system 34 as a single record.

It should be understood that the network 32 may be connected to the patient support apparatus 12 through a wired data link, or the network connection may be a wireless data link.

Although certain illustrative embodiments have been described in detail above, variations and modifications exist.

## Claims

1. A method for transferring medical data from a wireless sensor to a health information system, the method comprising receiving medical data from the wireless sensor (14), determining an identity of the wireless sensor (14), determining a nature of the received medical data; and transmitting the received medical data to the health information system (34) subsequent to determining the nature of the received medical data, **characterized in that** the method is for transferring medical data from a wireless sensor to a health information system using a patient support apparatus (12) **in that** the method comprises wirelessly transferring, with the patient support apparatus (12), a supply of power to the wireless sensor (14) to initiate operation of the wireless sensor (14), the medical data from the wireless sensor (14) being received with the patient support apparatus (12) while wirelessly transferring the supply of power to the wireless sensor (14), the identity of the wireless sensor (14) being determined on the patient support apparatus (12), and the nature of the received medical data being determined with the patient support apparatus (12); **in that** the method further comprises associating the received medical data with the patient support apparatus (12), and **in that** the received medical data and data identifying the associated patient support apparatus (12) are transmitted from the patient support apparatus (12) to the health information system (34) in response to determining the nature of the received medical data.

2. The method of claim 1, wherein receiving the medical data comprises receiving the medical data from wireless communication circuitry (18).

3. The method of either claim 1 or claim 2, wherein receiving the medical data from the wireless sensor (14) is in response to establishing a wireless communication connection between the patient support apparatus (12) and the wireless sensor (14).

4. The method of any preceding claim, wherein receiving the medical data is in response to transmitting, from the patient support apparatus (12), a request for the medical data.

5. The method of any preceding claim, wherein determining the identity of the wireless sensor (14) comprises performing, on the patient support apparatus (12), a handshaking protocol with the wireless sensor (14).

6. The method of claim 5, wherein performing the handshaking protocol comprises receiving, with the patient support apparatus (12), a unique identifier of the wireless sensor (14) from the wireless sensor (14).

7. The method of any preceding claim, wherein determining the nature of the received medical data comprises receiving identifying information associated with the medical data from the wireless sensor (14).

8. The method of any preceding claim, wherein transmitting the received medical data and the data identifying the associated patient support apparatus (12) comprises transmitting an alert message to the hospital information system.

9. The method of claim 8, wherein transmitting the alert message comprises transmitting an alert message to a nurse's station using a nurse call cable.

10. The method of claim 8, wherein transmitting the alert message comprises wirelessly transmitting the alert message.

11. The method of any preceding claim, further comprising analyzing, on the patient support apparatus (12), the received medical data to determine whether a health condition is met, the health condition and associated medical data criteria having been pre-established on the patient support apparatus (12).

12. The method of claim 11, further comprising performing a non-therapeutic function using the patient support apparatus (12) in response to the received medical data satisfying the health condition.

13. The method of claim 12, wherein performing the non-therapeutic function comprises at least one of: automatically performing a mechanical function and transmitting an alarm to the hospital information system.

14. The method of any preceding claim, further comprising transmitting, from the patient support apparatus (12) to the wireless sensor (14), instructions to update the wireless sensor (14) in response to receiving the instructions from the hospital information system.

15. A controller (16) of a patient support apparatus comprising a processor (26),and a memory (28) having stored therein a plurality of instructions that when executed by the processor cause the controller to implement the method of claim 1.

## Patentansprüche

1. Ein Verfahren zur Übertragung von medizinischen Daten von einem drahtlosen Sensor an ein Gesundheitsinformationssystem, wobei das Verfahren den Empfang medizinischer Daten von einem drahtlosen Sensor (14), die Ermittlung der Identität des drahtlosen Sensors (14), die Ermittlung der Art der empfangenen medizinischen Daten und nach der Ermittlung der Art der empfangenen medizinischen Daten die Übermittlung der medizinischen Daten an das Gesundheitsinformationssystem (34) umfasst, **dadurch gekennzeichnet, dass** das Verfahren für die Übertragung von medizinischen Daten von einem drahtlosen Sensor an ein Gesundheitsinformationssystem mittels einer Vorrichtung zur Patientenlagerung (12) ist, dass das Verfahren drahtloses Übertragen, mit der Vorrichtung zur Patientenlagerung (12), einer Stromversorgung des drahtlosen Sensors (14) umfasst, um den drahtlosen Sensor (14) in Betrieb zu setzen, wobei die medizinischen Daten von dem drahtlosen Sensor (14) mit der Vorrichtung zur Patientenlagerung (12) empfangen werden, während die Stromversorgung des drahtlosen Sensors (14) drahtlos übertragen wird, die Identität des drahtlosen Sensors (14) an der Vorrichtung zur Patientenlagerung (12) ermittelt wird und die Art der empfangenen medizinischen Daten mit der Vorrichtung zur Patientenlagerung (12) ermittelt wird; dass das Verfahren ferner das Zuordnen der empfangenen medizinischen Daten zu der Vorrichtung zur Patientenlagerung (12) umfasst, und dass die empfangenen medizinischen Daten und Daten, die die zugeordnete Vorrichtung zur Patientenlagerung (12) identifizieren, von der Vorrichtung zur Patientenlagerung (12) an das Gesundheitsinformationssystem (34) als Reaktion auf die Ermittlung der Art der empfangenen medizinischen Daten übertragen werden.

2. Das Verfahren nach Anspruch 1, wobei das Empfangen der medizinischen Daten das Empfangen der medizinischen Daten von einem drahtlosen Kommunikationsschaltkreis (18) umfasst.

3. Das Verfahren nach entweder Anspruch 1 oder Anspruch 2, wobei das Empfangen der medizinischen Daten von dem drahtlosen Sensor (14) eine Reaktion auf das Herstellen einer drahtlosen Kommunikationsverbindung zwischen der Vorrichtung zur Patientenlagerung (12) und dem drahtlosen Sensor (14) ist.

4. Das Verfahren nach einem der vorstehenden Ansprüche, wobei das Empfangen der medizinischen Daten eine Reaktion auf die Übermittlung einer Anforderung der medizinischen Daten durch die Vorrichtung zur Patientenlagerung (12) ist.

5. Das Verfahren nach einem der vorstehenden Ansprüche, wobei die Ermittlung der Identität des drahtlosen Sensors (14) umfasst, dass an der Vorrichtung zur Patientenlagerung (12) ein Handshake-Protokoll mit dem drahtlosen Sensor (14) durchgeführt wird.

6. Das Verfahren nach Anspruch 5, wobei das Durchführen des Handshake-Protokolls umfasst, dass mit der Vorrichtung zur Patientenlagerung (12) eine eindeutige Kennung des drahtlosen Sensors (14) von dem drahtlosen Sensor (14) empfangen wird.

7. Das Verfahren nach einem der vorstehenden Ansprüche, wobei die Ermittlung der Art der empfangenen medizinischen Daten umfasst, dass Identifizierungsdaten, die mit den medizinischen Daten von dem drahtlosen Sensor (14) zusammenhängen, empfangen werden.

8. Das Verfahren nach einem der vorstehenden Ansprüche, wobei das Übermitteln der empfangenen medizinischen Daten und der Daten, welche die zugeordnete Vorrichtung zur Patientenlagerung (12) identifizieren, umfasst, dass eine Warnmeldung an das Krankenhausinformationssystem übermittelt wird.

9. Das Verfahren nach Anspruch 8, wobei das Übermitteln der Warnmeldung umfasst, dass eine Warnmeldung an das Stationszimmer mittels eines Schwesternrufkabels übermittelt wird.

10. Das Verfahren nach Anspruch 8, wobei das Übermitteln der Warnmeldung umfasst, dass eine Warnmeldung drahtlos übermittelt wird.

11. Das Verfahren nach einem der vorstehenden Ansprüche, ferner umfassend, dass an der Vorrichtung zur Patientenlagerung (12) die empfangenen medizinischen Daten analysiert werden, um zu ermitteln, ob ein Gesundheitszustand erfüllt ist, wobei der Gesundheitszustand und die damit zusammenhängenden medizinischen Datenkriterien vorher an der Vorrichtung zur Patientenlagerung (12) festgelegt wurden.

12. Das Verfahren nach Anspruch 11, ferner umfassend, dass eine nichttherapeutische Funktion mittels der Vorrichtung zur Patientenlagerung (12) als Reaktion auf die empfangenen medizinischen Daten, die dem Gesundheitszustand gerecht werden, ausgeführt wird.

13. Das Verfahren nach Anspruch 12, wobei die Ausführung der nichttherapeutischen Funktion mindestens eines umfasst: automatisches Ausführen einer mechanischen Funktion und Übermitteln eines Alarms an das Krankenhausinformationssystem.

14. Das Verfahren nach einem der vorstehenden Ansprüche, ferner umfassend, dass von der Vorrichtung zur Patientenlagerung (12) an den drahtlosen Sensor (14) Anweisungen zur Aktualisierung des drahtlosen Sensors (14) als Reaktion auf den Empfang von Anweisungen vom Krankenhausinformationssystem übermittelt werden.

15. Eine Steuereinheit (16) einer Vorrichtung zur Patientenlagerung, umfassend einen Prozessor (26) und einen Speicher (28), in dem eine Vielzahl von Anweisungen gespeichert ist, die bei Ausführung durch den Prozessor die Steuereinheit veranlassen, das Verfahren nach Anspruch 1 umzusetzen.

## Revendications

1. Procédé pour le transfert de données médicales depuis un capteur sans fil vers un système d'informations de santé, le procédé comprenant la réception de données médicales depuis le capteur sans fil (14), la détermination d'une identité du capteur sans fil (14), la détermination d'une nature des données médicales reçues ; et la transmission des données médicales reçues au système d'informations de santé (34) suite à la détermination de la nature des données médicales reçues, **caractérisé en ce que** le procédé est destiné au transfert de données médicales depuis un capteur sans fil vers un système d'informations de santé à l'aide d'un appareil de support de patient (12), **en ce que** le procédé comprend le transfert sans fil, avec l'appareil de support de patient (12), d'une alimentation électrique au capteur sans fil (14) pour initier le fonctionnement du capteur sans fil (14), les données médicales provenant du capteur sans fil (14) étant reçues avec l'appareil de support de patient (12) tout en transférant sans fil l'alimentation électrique au capteur sans fil (14), l'identité du capteur sans fil (14) étant déterminée sur l'appareil de support de patient (12), et la nature des données médicales reçues étant déterminée avec l'appareil de support de patient (12) ; **en ce que** le procédé comprend en outre l'association des données médicales reçues avec l'appareil de support de patient (12), et **en ce que** les données médicales reçues et les données identifiant l'appareil de support de patient (12) associé sont transmises depuis l'appareil de support de patient (12) vers le système d'informations de santé (34) en réponse à la détermination de la nature des données médicales reçues.

2. Procédé selon la revendication 1, dans lequel la réception des données médicales comprend la réception des données médicales depuis des circuits de communication sans fil (18).

3. Procédé selon la revendication 1 ou la revendication 2, dans lequel la réception des données médicales depuis le capteur sans fil (14) se fait en réponse à l'établissement d'une connexion de communication sans fil entre l'appareil de support de patient (12) et le capteur sans fil (14).

4. Procédé selon une quelconque revendication précédente, dans lequel la réception des données médicales se fait en réponse à la transmission, depuis l'appareil de support de patient (12), d'une demande des données médicales.

5. Procédé selon une quelconque revendication précédente, dans lequel la détermination de l'identité du capteur sans fil (14) comprend l'exécution, sur l'appareil de support de patient (12), d'un protocole de liaison avec le capteur sans fil (14).

6. Procédé selon la revendication 5, dans lequel l'exécution du protocole de liaison comprend la réception, avec l'appareil de support de patient (12), d'un identifiant unique du capteur sans fil (14) depuis le capteur sans fil (14).

7. Procédé selon une quelconque revendication précédente, dans lequel la détermination de la nature des données médicales reçues comprend la réception d'informations d'identification associées aux données médicales provenant du capteur sans fil (14).

8. Procédé selon une quelconque revendication précédente, dans lequel la transmission des données médicales reçues et des données identifiant l'appareil de support de patient (12) associé comprend la transmission d'un message d'alerte au système d'informations hospitalier.

9. Procédé selon la revendication 8, dans lequel la transmission du message d'alerte comprend la transmission d'un message d'alerte au poste de personnel infirmier à l'aide d'un câble d'appel de personnel infirmier.

10. Procédé selon la revendication 8, dans lequel la transmission du message d'alerte comprend la transmission sans fil du message d'alerte.

11. Procédé selon une quelconque revendication précédente, comprenant en outre l'analyse, sur l'appareil de support de patient (12), des données médicales reçues pour déterminer si un état de santé satisfait à l'état de santé et aux critères de données médicales associées ayant été prédéfinis sur l'appareil de support de patient (12).

12. Procédé selon la revendication 11, comprenant en outre l'exécution d'une fonction non thérapeutique à l'aide de l'appareil de support de patient (12) en réponse aux données médicales reçues satisfaisant à l'état de santé.

13. Procédé selon la revendication 12, dans lequel l'exécution de la fonction non thérapeutique comprend au moins une action parmi : effectuer automatiquement une fonction mécanique et transmettre une alarme au système d'informations hospitalier.

14. Procédé selon une quelconque revendication précédente, comprenant en outre la transmission, depuis l'appareil de support de patient (12) vers le capteur sans fil (14), d'instructions pour mettre à jour le capteur sans fil (14) en réponse à la réception des instructions provenant du système d'informations hospitalier.

15. Dispositif de commande (16) d'un appareil de support de patient comprenant un processeur (26), et une mémoire (28) ayant stocké en son sein une pluralité d'instructions qui, lorsqu'elles sont exécutées par le processeur, provoquent la mise en oeuvre par le dispositif de commande du procédé selon la revendication 1.
